# EUROPEAN PATENT APPLICATION

(11) **EP 2 980 054 A1**
(43) Date of publication of application: **03.02.2016**
(21) Application number: 14191703.9
(22) Date of filing: 04.11.2014
(51) Int. Cl.: C07C 29/76, C07C 31/08

(54) **METHOD OF ANHYDROUS BIOETHANOL PRODUCTION**

(30) Priority: 28.07.2014 PL 40900114
(71) Applicant: Politechnika Lubelska, 20-618 Lublin (PL)
(72) Inventor: Paw Owski, Artur, 20-710 Lublin (PL); Paw Owska, Ma gorzata, 20-710 Lublin (PL); Kwiatkowski, Zygmunt, 21-003 Ciecierzyn (PL); Kujawska, Justyna, 20-861 Lublin (PL); Cel, Wojciech, 97-300 Piotrków Trybunalski (PL)
(74) Representative: Kaminski, Piotr

(57) **Abstract**

Invention relates to a method of producing anhydrous bioethanol wherein rectified spirit is intensively mixed with a brown coal ash in the weight ratio of 100:70±5 over the period of 20-30 minutes and afterwards precipitated sediment is separated by a filter press or a centrifuge.

## Description

The invention is a method of producing anhydrous bioethanol.

According to the European Commission regulation, in 2020 the EU Member States will have to use 10% of biofuels in transport sector. In Poland, biodiesel and bioethanol are manufactured. In the process of fermentation approximately 10% solution of ethanol is produced which is condensed through distillation into the rectified spirit containing 96.5% alcohol by volume.

Ethanol to be used as a biofuel must be in a form of an anhydrous alcohol. The method of dehydrating 95.6% rectified spirit through distillation with the addition of benzene, diisopropyl ester or cyclohexane, which form a distillable tri-azeotrope is already known. The disadvantages of this method are the necessity of using the above-mentioned toxic compounds and relatively high energy consumption.

Another method, presented in patent US4769112 relies on removal of water from the rectified spirit and transforming it into anhydrous ethanol by means of liquid carbon dioxide. The drawback of this method is the complicated application of carbon dioxide and therefore it is rarely used in the industry.

Yet another method currently in use employs membranes for dewatering of ethanol to 99.9% bioethanol by volume. Type 3A molecular sieves as well as biosorbents are also utilized in the process of dehydrating rectified spirit. These methods are seldom used due to limited access to suitable membranes, sieves and biosorbents.

Polish patent application P.319709 presents the method of producing an anhydrous ethanol - particularly from neutral grain spirits - through adsorption of water vapour on type 4A molecular sieve, which is then regenerated by heating, reducing pressure and purging with a carrier gas.

The Polish patent P 192413 presents anhydrous alcohol production through rectification with use of azeotropic agent. It is based on the rectification of ethanol-water solution by means of methyl tert-butyl ether used in proportion of at least 0.05 parts per mass for 1 part of mass of water.

The essence of method of anhydrous bioethanol production is that 95.6% rectified ethanol is intensively mixed with the brown coal ash in the weight ratio of 100:70±5 over the period of 20-40 minutes. The precipitated sediment is then separated by a filter press or a centrifuge.

The advantage of the invention is utilisation of the waste ash produced during the combustion of brown coal in combined heat and power plants.

In the method of producing anhydrous bioethanol according to invention the rectified ethanol and the brown coal ash obtained through combustion are mixed intensively in the 100:70±5 weight ratio over the period of 20-30 minutes. The precipitated sediment is then separated by a filter press or a centrifuge.

### Example 1:

100 g of a rectified spirit containing 95.6% ethanol by volume was intensively mixed with 70 grams of brown coal ash for 20 minutes. The precipitated sediment was then separated in a centrifuge. The hydration of bioethanol amounted to 99.8 ± 0.1%.

### Example 2:

100 g of rectified spirit containing 95.6% ethanol by volume was intensively mixed with 75 grams of brown coal ash for 20 minutes. The precipitated sediment was then separated in a centrifuge. The hydration of bioethanol amounted to 99.9 ± 0.1%.

## Claims

1. A method of producing anhydrous bioethanol **characterized in that** rectified spirit is intensively mixed with a brown coal ash in the weight ratio of 100:70±5 over the period of 20-30 minutes and afterwards precipitated sediment is separated by a filter press or a centrifuge.
